# EUROPÄISCHE PATENTSCHRIFT

(11) **EP 0 884 063 B1**
(45) Veröffentlichungstag und Bekanntmachung des Hinweises auf die Patenterteilung: **28.04.2004**
(21) Anmeldenummer: 97201746.1
(22) Anmeldetag: 10.06.1997
(51) Int. Cl.: A61M 25/00, A61M 25/01, A61M 25/10

(54) **Kathetersystem**
Catheter system
Système de cathéter

(43) Veröffentlichungstag der Anmeldung: 16.12.1998
(73) Patentinhaber: Schneider (Europe) GmbH, 8180 Bülach (CH)
(72) Erfinder: Delaloye, Stéphane, 8180 Bülach (CH)
(74) Vertreter: Kiliaridis, Constantin

(56) Entgegenhaltungen:
- WO-A-95/32011
- DE-C- 3 935 256
- US-A- 5 364 376
- US-A- 5 458 605

## Beschreibung

Die Erfindung bezieht sich auf ein Kathetersystem zum schnellen Auswechseln über einen Führungsdraht mit einem flexiblen, länglichen Innenkatheter, der ein proximales Ende und ein distales Ende sowie ein Führungsdrahtlumen aufweist, welches sich von einer Einführöffnung am distalen Ende bis zu einer seitlichen Durchgangsöffnung distal vom proximalen Ende erstreckt, und einem flexiblen, rohrförmigen Aussenkatheter, in dem der Innenkatheter axial verschiebbar angeordnet ist und der eine seitliche Austrittsöffnung für den Führungsdraht aufweist.

Kathetersysteme mit zwei ineinander angeordneten und relativ zueinander verschiebbaren Kathetern haben bei Eingriffen in Körperhohlräume, wie etwa in Blutgefässe, Atemwege oder die Speiseröhre, vielseitige Anwendungsmöglichkeiten. Üblicherweise wird das Kathetersystem längs eines vorplatzierten Führungsdrahtes durch ein Körperlumen bis an die gewünschte Stelle eingeführt, um dort zum Einsatz zu kommen.

Beispielsweise zeigt die US 4,655,746 ein Kathetersystem, bei dem Innen- und Aussenkatheter als Ballonkatheter ausgebildet sind, so dass ein durch die Ballons abgegrenzter Gefässabschnitt gegenüber dem restlichen Gefässsystem abgedichtet werden kann. Der Ballonabstand und damit die Länge des abzudichtenden Gefässabschnitts kann durch Verschieben des Aussenkatheters relativ zum Innenkatheter eingestellt werden. Über ein dafür vorgesehenes Lumen wird in den abgedichteten Gefässabschnitt ein Behandlungsmittel infundiert oder Flüssigkeit daraus abgesaugt. Das Führungsdrahtlumen erstreckt sich bei diesem Kathetersystem über die gesamte Länge des Innenkatheters. Im Falle eines Katheterwechsels bei liegenbleibendem Führungsdraht ist entweder ein sehr langer Führungsdraht oder das Anbringen einer Verlängerung erforderlich. Beides ist sehr zeitaufwendig und stellt eine zusätzliche Belastung für den Patienten dar. Ferner muss bei einem solchen Eingriff eine zweite Person assistieren und es ist eine grosse Fläche um die Punktionsöffnung steril zu halten.

Zur Überwindung der vorgenannten Nachteile wurden Kathetersysteme entwckelt, bei denen sich das Führungsdrahtlumen nur über einen kurzen, distalen Bereich erstreckt und der Führungsdraht zum grössten Teil seiner Länge neben dem Kathetersystem nach proximal geführt wird. Der Führungsdraht ragt nur etwa um die Länge des Führungsdrahtlumens aus dem Körper, wobei ein schneller Katheterwechsel ohne Anbringen einer Verlängerung möglich ist.

Aus der EP 0 505 686 B1 ist ein Kathetersystem der eingangs genannten Art bekannt, bei dem der Innenkatheter als schnell auswechselbarer Ballonkatheter mit einem vormontierten Stent ausgebildet ist, während der Aussenkatheter als Schutzhülle beim Einführen des Stents dient. Der Führungsdraht verlässt das Kathetersystem proximal durch zwei aufeinander ausgerichtete Öffnungen in Innen- und Aussenkatheter. Zum Einführen des Kathetersystems werden die beiden Öffnungen zumindest teilweise zur Deckung gebracht, damit das Kathetersystem sich längs des Führungsdrahts bewegen lässt. Zum Aufweiten des Stents wird der Aussenkatheter längs des Innenkatheters zurückgezogen, wodurch sich die Führungsdrahtöffnungen gegeneinander verschieben und der Führungsdraht zwischen den Öffnungen in einem ringförmigen Lumen zwischen Innen- und Aussenkatheter verläuft. Nach erfolgter Stentimplantation kann zunächst der der Aussenkatheter zurückgezogen werden, der zu diesem Zweck einen sich von der Führungsdrahtöffnung bis zum distalen Ende des Aussenkatheters erstreckenden Schlitz aufweist. Anschliessend wird der Ballonkatheter in bekannter Weise längs des Führungsdrahtes aus dem Körper gezogen. In der Handhabung eines solchen Kathetersystems etwa im Gefässsytem eines Patienten können sich Innen- und Aussenkatheter insbesondere im distalen Bereich des Kathetersystems gegeneinander verdrehen, wobei sich die gegenseitige Lage der Führungsdrahtöffnungen verändert. Beim Einführen oder Zurückziehen des Kathetersystems führt dies zu unerwünschtem Einklemmen des Führungsdrahts im Bereich der Öffnungen, was ein schnelles und gleichmässiges Bewegen eines solchen Kathetersystems erheblich behindert. Ausserdem können die Führungsdrahtöffnungen beim Verschieben des Aussenkatheters längs des Innenkatheters ihre gegenseitige Ausrichtung verlieren und es müssen beim Zurückziehen gegebenenfalls zusätzliche Reibungskräfte zwischen Führungsdraht und Öffnungsrand überwunden werden. Schliesslich kann der Führungsdraht beim Zurückziehen des Aussenkatheters in den Schlitz gedrückt und dort eingeklemmt werden.

Ein ebenfalls schnell austauschbares Kathetersystem zeigt die WO 94/15549. Am distalen Ende des Kathetersystems ist zwischen Innen- und Aussenkatheter ein selbstexpandierender Stent in seinem komprimierten Zustand gespannt, der durch Zurückziehen des Aussenkatheters relativ zum Innenkatheter freigesetzt wird. Der Führungsdraht tritt proximal aus dem Kathetersystem durch eine seitliche Öffnung im Innenkatheter und einen Schlitz im Aussenkatheter. An die Öffnung im Innenkatheter schliesst sich proximal eine längliche Vertiefung an, in die sich der Führungsdraht beim Zurückziehen des Aussenkatheters legen kann. Dadurch wird der Führungsdraht ohne starke Verbiegung sanft aus dem Kathetersystem geführt. Der Schlitz im Aussenkatheter ist jedoch so schmal, dass beim Verschieben des Kathetersystems längs des Führungsdrahtes, aber auch beim Zurückziehen des Aussenkatheters erhebliche Reibungskräfte auftreten. Des weiteren leidet das Kathetersystem unter dem Nachteil, dass der Schlitz und die Vertiefung den Querschnitt und damit die Knickstabilität des Kathetersystems verringern, insbesondere wenn sich Schlitz und Vertiefung bis zum proximalen Ende des Kathetersystems erstrecken.

US patent 5,364,376 offenbart ein Kathetersystem zum schnellen Auswechseln über einen Führungsdraht mit einem flexiblen, länglichen Innenkatheter, der ein proximales Ende und ein distales Ende sowie ein Führungsdrahtlumen aufweist, welches sich von einer Einführöffnung am distalen Ende bis zu einer seitlichen Durchgangsöffnung distal vom proximalen Ende erstreckt, und einem flexiblen, rohrförmigen Aussenkatheter, in dem der Innenkatheter axial verschiebbar angeordnet ist und der eine seitliche Austrittsöffnung für den Führungsdraht aufweist, wobei an Innenkatheter und Aussenkatheter Sicherungsmittel vorgesehen sind, die ein Verdrehen des Aussenkatheters relativ zum Innenkatheter verhindern, wodurch die Durchgangsöffnung und die Austrittsöffnung zueinander ausgerichtet bleiben.

Der Nachteil dieses Katheters ist vor allem einen steifen Katheter auszubilden, welcher in engen Kurven ein Problem darstellt.

Der Erfindung liegt daher die Aufgabe zugrunde, eine Kathetersystem der eingangs genannten Art anzugeben, welches gleichmässig und leicht längs eines Führungsdrahtes bewegbar ist und bei dem sich der Aussenkatheter einfach relativ zum Innenkatheter zurückziehen lässt.

Die Aufgabe wird gemäss der Erfindung gelöst durch ein Kathetersystem der eingangs genannten Art mit den Merkmalen des kennzeichnenden Teils des Patentanspruchs 1. Wenn am Innenkatheter und Aussenkatheter Sicherungsmittel vorgesehen sind, die ein Verdrehen des Aussenkatheters relativ zum Innenkatheter verhindern, wodurch die Durchgangsöffnung und die Austrittsöffnung zueinander ausgerichtet bleiben, wird dem Führungsdraht eine grosse Öffnungsfläche zum Austreten aus dem Kathetersystem bereitgstellt. Ein Einklemmen des Führungsdrahtes zwischen Durchgangs- und Austrittsöffnung wird dadurch sowohl beim Vorschieben oder Zurückziehen des gesamten Kathetersystems längs des Führungsdrahtes als auch beim Verschieben des Aussenkatheters relativ zum Innenkatheter vermieden. Ein erfindungsgemässes Kathetersystem weist damit verbesserte Gleiteigenschaften auf, die seine Handhabbarkeit im praktischen Einsatz erleichtert.

In einer vorteilhaften Ausgestaltung der Erfindung werden die Sicherungsmittel formschlüssig durch eine Vertiefung in der Aussenwand des Innenkatheters und durch mindestens einen in die Vertiefung eingreifenden Vorsprung auf der Innenwand des Aussenkatheters gebildet. Dadurch ist eine einfach herstellbare Verdrehsicherung zwischen Innenkatheter und Aussenkatheter geschaffen, die eine axiale Relativverschiebung von Aussen- und Innenkatheter nicht beeinträchtigt. Die Sicherungsmittel sind derart in das Kathetersystem integriert, dass durch sie keine Profilvergrösserung in Kauf genommen werden muss. Ohne den Schutzbereich zu verlassen, kann umgekehrt die Vertiefung auch im Aussenkatheter und die Vorsprünge am Innenkatheter angebracht sein; auch eine Mischform ist denkbar.

In einer bevorzugten Ausführungsform der Erfindung ist die Vertiefung länglich mit axialer Ausrichtung ausgebildet. Die Verdrehsicherung erfüllt hier zusätzlich die Aufgabe, den Aussenschaft beim Zurückziehen relativ zum Innenschaft zu führen. Darüber hinaus kann durch diese Massnahme eine distale und proximale Endposition des Aussenschaftes relativ zum Innenschaft definiert werden.

Der Vorsprung ist in seiner axialen Ausdehnung vorzugsweise klein im Vergleich zur Länge der Vertiefung. Dadurch wird errreicht, dass die Länge der Vertiefung möglichst klein bleiben kann, nämlich etwa in der Grössenordnung, die sich aus der praktischen Anwendung ergibt - also zum Beispiel die Länge eines maximal durch zwei Ballons abzudichtenden Gefässabschnitts oder die verwendete Stentlänge. Ausserdem wird die Reibungsfläche zwischen Vorsprung und Vertiefung verringert, was beim Verschieben des Aussenkatheters zu einer einfachen Handhabung eines erfindungsgemässen Kathetersystems beiträgt.

In einer vorteilhaften Ausführungsform der Erfindung ist die Durchgangsöffnung am distalen Ende der Vertiefung angeordnet. In dieser Anordnung kann sich der Führungsdraht beim Zurückziehen des Aussenkatheters in die Vertiefung legen, wodurch eine starke Krümmung des Führungsdrahtes beim Verlassen des Kathetersystems vermieden wird. Zusätzlich erreicht man, dass der Führungsdraht im Bereich der Vertiefung keine Querschnittsfläche innerhalb des Innenkatheters beansprucht. Hierdurch kann der Gesamtquerschnitt des Kathetersystems klein gehalten werden.

In einer bevorzugten Ausgestaltung der Erfindung führt die Austrittsöffnung durch den Vorsprung. Die Verdrehsicherung bzw. Führung ist hier besonders effektiv, da sie direkt auf den Verlauf des Führungsdrahtes wirken, indem sie die Austrittsöffnung und die Durchgangsöffnung in jeder Katheterstellung aufeinander ausgerichtet halten. Die Ausgestaltung hat ausserdem fertigungstechnische Vorteile, da Austrittsöffnung und Vorsprung mit einem einzigen Fertigungsgang hergestellt werden können.

Die Durchgangsöffnung und die Austrittsöffnung eines erfindungsgemässen Kathetersystems sind in vorteilhafter Weise mit Führungshilfen zum distalen Einführen des Führungsdrahtes versehen. Der am distalen Ende des Kathetersystems eingefädelte Führungsdraht wird dadurch beim Vorschieben des Kathetersystems ohne zusätzliche Manipulation durch die Durchgangsöffnung und die Austrittsöffnung aus dem Führungsdrahtlumen geführt. Ein erfindungsgemässes Kathetersystem kann dadurch schnell und problemlos über einen vorplazierten Führungsdraht eingeführt werden.

Weitere Vorteile eines erfindungsgemässen Kathetersystems ergeben sich aus einem bevorzugten Ausführungsbeispiel, das im folgenden anhand der Zeichnung näher beschrieben wird, in deren
- FIG. 1: ein Abschnitt des Kathetersystems im Längsschnitt und in
- FIG. 2: ein Querschnitt des Kathetersystems längs der Schnittlinie A-A aus FIG. 1 dargestellt ist.

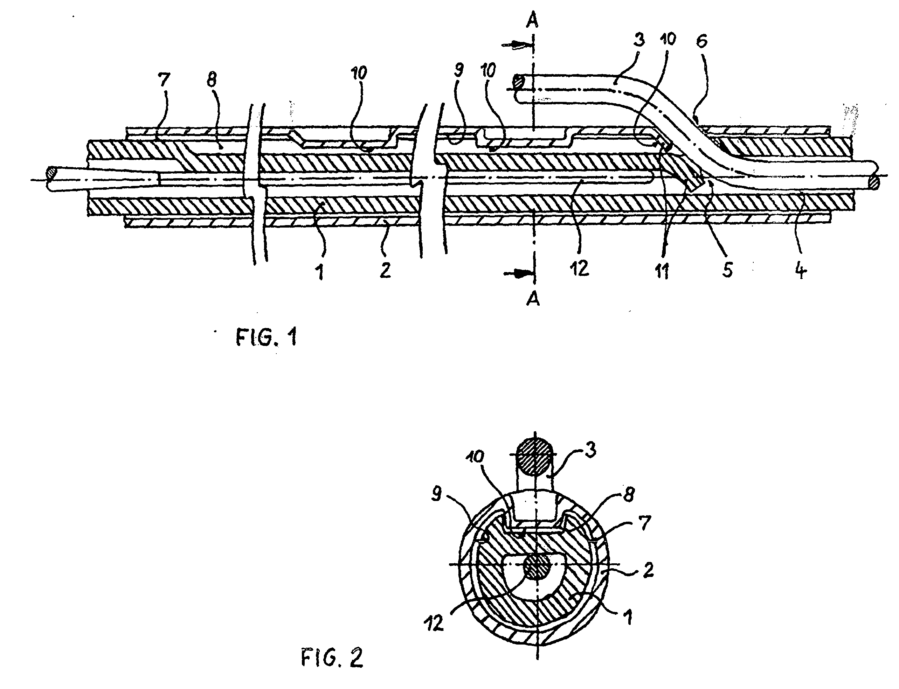

FIG. 1 und FIG. 2 veranschaulichen ein erfindungsgemässes Kathetersystem mit einem flexiblen, länglichen Innenkatheter 1 und einem flexiblen, rohrförmigen Aussenkatheter 2, welches zum schnellen Auswechseln über einen Führungsdraht 3 geeignet ist. Dazu erstreckt sich das Führungsdrahtlumen 4 von einer Einführöffnung (nicht dargestellt) am distalen Ende (nicht dargestellt) des Innenkatheters 1 bis zu einer Durchgangsöffnung 5, welche distal vom proximalen Ende (nicht dargestellt) des Innenkatheters 1 liegt. Der Aussenkatheter 2 weist eine seitliche Austrittsöffnung 6 für den Führungsdraht 3 auf, ab welcher der Führungsdraht 3 nach proximal neben dem Kathetersystem verläuft

Der Innenkatheter 1 weist an seiner Aussenwand 7 eine längliche, axial ausgerichtete Vertiefung 8 auf, in welche an der Innenwand 9 angeordnete Vorsprünge 10 eingreifen. Dadurch ergibt sich eine formschlüssige Verdrehsicherung zwischen Innenkatheter 1 und Aussenkatheter 2, welche die Austrittsöffnung 6 und die Durchgangsöffnung 5 sowohl beim Verschieben des gesamten Kathetersystems längs des Führungsdrahtes 3 als auch beim Verschieben des Aussenkatheters 2 relativ zum Innenkatheter 1 aufeinander ausgerichtet hält. Somit wird dem Führungsdraht 3 in allen Positionen eine möglichst grosse Fläche für den Austritt aus dem Kathetersystem zur Verfügung gestellt und die Reibung zwischen Führungsdraht 3 und Durchgangsöffnung 5 beziehungsweise Austrittsöffnung 6 klein gehalten. Die Handhabung eines erfindungsgemässen Kathetersystems wird erheblich erleichtert, da lagebedingte Reibungswiderstände nicht überwunden werden müssen. Zur weiteren Erleichterung in der Handhabung sind die Durchgangsöffnung 5 und die Austrittsöffnung 6 mit Führungshilfen 11 versehen, die den von distal durch die Einführöffnung in das Führungsdrahtlumen 4 eingefädelten Führungsdraht 3 ohne zusätzliche Manipulationen beim Vorschieben des Kathetersystems seinen Weg durch Durchgangsöffnung 5 und Austrittsöffnung 6 finden lassen.

Vertiefung 8 und Vorsprung 10 können beispielsweise einfach durch thermische Verformung mittels Formwerkzeugen in Innenkatheter 1 und Aussenkatheter 2 eingeprägt werden. Die Führungshilfen 11 lassen sich ebenfalls ohne zusätzlichen Aufwand beim Einarbeiten von Durchgangsöffnung 5 und Austrittsöffnung 6 herstellen, indem sie durch den Materialüberstand an den Bohrungsrändern gebildet werden.

Im Innenkatheter 1 verläuft ein sich in distale Richtung verjüngender Versteifungsdraht 12, der zur Stützung des Abschnittes, in dem die Vertiefung 8 angeordnet ist, beiträgt.

### Bezugszeichenliste

- 1: Innenkatheter
- 2: Aussenkatheter
- 3: Führungsdraht
- 4: Führungsdrahtlumen
- 5: Durchgangsöffnung
- 6: Austrittsöffnung
- 7: Aussenwand
- 8: Vertiefung
- 9: Innenwand
- 10: Vorsprung
- 11: Führungshilfen
- 12: Versteifungsdraht

## Patentansprüche

1. Kathetersystem zum schnellen Auswechseln über einen Führungsdraht (3) mit einem flexiblen, länglichen Innenkatheter (1), der ein proximales Ende und ein distales Ende sowie ein Führungsdrahtlumen (4) aufweist, welches sich von einer Einführöffnung am distalen Ende bis zu einer seitlichen Durchgangsöffnung (5) distal vom proximalen Ende erstreckt, und einem flexiblen, rohrförmigen Aussenkatheter (2), in dem der Innenkatheter (1) axial verschiebbar angeordnet ist und der eine seitliche Austrittsöffnung (6) für den Führungsdraht (3) aufweist,
wobei an Innenkatheter (1) und Aussenkatheter (2) Sicherungsmittel vorgesehen sind, die ein Verdrehen des Aussenkatheters (2) relativ zum Innenkatheter (1) verhindern, wodurch die Durchgangsöffnung (5) und die Austrittsöffnung (6) zueinander ausgerichtet bleiben, **dadurch gekennzeichnet, dass** die Sicherungsmittel kurz im Vergleich zur axialen Länge der Katheter sind.

2. Kathetersystem nach Anspruch 1,
**dadurch gekennzeichnet, dass** die Sicherungsmittel formschlüssig durch eine Vertiefung (8) in der Aussenwand (7) des Innenkatheters (1) und durch mindestens einen in die Vertiefung (8) eingreifenden Vorsprung (10) auf der Innenwand (9) des Aussenkatheters (2) gebildet werden.

3. Kathetersystem nach Anspruch 2,
**dadurch gekennzeichnet, dass** die Vertiefung (8) länglich mit axialer Ausrichtung ausgebildet ist.

4. Kathetersystem nach Anspruch 2 oder 3,
**dadurch gekennzeichnet, dass** der Vorsprung (10) in seiner axialen Ausdehnung klein ist im Vergleich zur Länge der Vertiefung (8).

5. Kathetersystem nach einem der Ansprüche 2 bis 4,
**dadurch gekennzeichnet, dass** die Durchgangsöffnung (5) am distalen Ende der Vertiefung (8) angeordnet ist.

6. Kathetersystem nach einem der Ansprüche 2 bis 5,
**dadurch gekennzeichnet, dass** die Austrittsöffnung (6) durch den Vorsprung (10) führt.

7. Kathetersystem nach einem der Ansprüche 1 bis 6,
**dadurch gekennzeichnet, dass** die Durchgangsöffnung (5) und die Austrittsöffnung (6) mit Führungshilfen (11) zum distalen Einführen des Führungsdrahtes (3) versehen sind.

## Claims

1. A catheter system for rapid exchange via a guide wire (3) with a flexible, elongated inner catheter (1), which has a proximal end and a distal end as well as a guide wire lumen (4), which extends from an insertion port on the distal end to a lateral through port (5) distal of the proximal end, and a flexible, tubular outer catheter (2), in which the inner catheter (1) is disposed axially displaceably and which has a lateral exit port (6) for the guide wire (3), wherein retaining means are provided on the inner catheter (1) and the outer catheter (2), which retaining means prevent twisting of the outer catheter (2) relative to the inner catheter (1), whereby the through port (5) and the exit port (6) remain aligned with each other, **characterised in that** said retaining means are short with respect to the axial length of said catheters.

2. The catheter system according to claim 1, **characterised in that** the retaining means are formed interlockingly by a depression (8) in the outer wall (7) of the inner catheter (1) and by at least one protrusion (10) on the inner wall (9) of the outer catheter (2) engaging in the depression (8).

3. The catheter system according to claim 2, **characterised in that** the depression (8) is elongated with axial alignment.

4. The catheter system according to claim 2 or 3, **characterised in that** the protrusion (10) is small in its axial dimension compared to the length of the depression (8).

5. The catheter system according to any one of claims 2 through 4, **characterised in that** the through port (5) is disposed on the distal end of the depression (8).

6. The catheter system according to any one of claims 2 through 5, **characterised in that** the exit port (6) passes through the protrusion (10).

7. The catheter system according to any one of claims 1 through 7, **characterised in that** the through port (5) and the exit port (6) are provided with guiding aids (11) for the distal insertion of the guide wire (3).

## Revendications

1. Système de cathéter à échange rapide sur un fil de guidage (3) avec un cathéter interne (1) flexible, allongé, qui comporte une extrémité proximale, une extrémité distale et une lumière pour fil de guidage (4), laquelle s'étend d'une ouverture d'introduction à l'extrémité distale jusqu'à une ouverture de passage latérale (5) située distalement par rapport à l'extrémité proximale, et un cathéter externe (2) flexible de forme tubulaire, dans lequel le cathéter interne (1) est arrangé déplaceable axialement et comprend une ouverture de sortie latérale pour le fil de guidage (3), dans lequel des moyens de retenue sont prévus sur le cathéter interne (1) et le cathéter externe (2), lesquels empêchent une rotation du cathéter externe relativement au cathéter interne (1), par lesquels l'ouverture de passage (5) et l'ouverture de sortie (6) restent alignées l'une par rapport à l'autre, **caractérisé en ce que** les moyens de retenue sont courts par rapport à la longueur axiale du cathéter.

2. Système de cathéter selon la revendication 1, **caractérisé en ce que** les moyens de retenue s'engagent l'un dans l'autre et sont formés par un creux (8) dans la paroi externe (7) du cathéter interne (1) et par au moins une partie en saillie (10) sur la paroi interne du cathéter externe (2) qui s'engage dans le creux (8).

3. Système de cathéter selon la revendication 2, **caractérisé en ce que** le creux (8) est formé en longueur avec un alignement axial.

4. Système de cathéter selon la revendication 2 ou 3, **caractérisé en ce que** la partie en saillie (10) est petite dans sa dimension axiale par rapport à la longueur du creux (8).

5. Système de cathéter selon l'une des revendications 2 à 4, **caractérisé en ce que** l'ouverture de passage (5) est arrangée à l'extrémité distale du creux (8).

6. Système de cathéter selon l'une des revendications 2 à 5, **caractérisé en ce que** l'ouverture de sortie (6) passe à travers la partie en saillie (10).

7. Système de cathéter selon l'une des revendications 1 à 6, **caractérisé en ce que** l'ouverture de passage (5) et l'ouverture de sortie (6) sont munies de moyens de guidage (11) pour l'introduction distale du fil de guidage (3).
